# EUROPEAN PATENT APPLICATION

(11) **EP 2 962 715 A1**
(43) Date of publication of application: **06.01.2016**
(21) Application number: 14756609.5
(22) Date of filing: 27.02.2014
(51) Int. Cl.: A61M 5/28, A61J 1/14, A61M 5/31, B65D 55/08, G09F 3/03

(54) **SYRINGE LABEL, AND MEDICINAL SOLUTION-FILLED SYRINGE USING SAME**

(30) Priority: 27.02.2013 JP 2013037261
(71) Applicant: Teva Pharma Japan Inc., Nagoya-shi, Aichi 453-0801 (JP)
(72) Inventor: IGARASHI, Masayuki, Ichinomiya-shi Aichi 491-0827 (JP); KOMURO, Chikara, Tokyo 115-8588 (JP); TAKEYAMA, Keisuke, Nagoya-shi Aichi 453-0801 (JP)
(74) Representative: Keseris, Denis
(86) International application number: PCT/JP2014/054794
(87) International publication number: WO 2014/133053

(57) **Abstract**

Slightly above a rupture-intended line 2, a peripheral surface of a projection portion 103a of a stopper cap 103 is pushed in a direction from an outer side to an inner side (in an arrow direction). The stopper cap 103 is pushed over on the principle of leverage in an oblique direction with, as a fulcrum P, the side (the back side) opposite in a circumferential direction to the side (the near side) at which a pushing force PW acts, so that an intermittent portion is ruptured due to bending of the flexible stopper cap 103. Then, a residual area Z1 remains at the syringe 101 side, and a separation area Z2 is separated together with the stopper cap 103 from the syringe 101 to perform unsealing.

Thus, a syringe label which allows for easy unsealing along the intermittent portion and with which an unsealed state is easily confirmed, and a drug solution-filled syringe using the label, are provided.

## Description

### FIELD OF THE INVENTION

The invention relates to a syringe label for sealing a stopper cap to a syringe (injector body) filled with a medical drug solution, and a drug solution-filled syringe sealed with the syringe label.

### BACKGROUND OF THE INVENTION

A shaft-shaped stopper cap is mounted into an opening of a tip portion of a syringe filled with a drug solution, and a heat-shrinkable resin film (shrink label) is wrapped around the tip portion of the syringe and a projection portion of the stopper cap. When the label is caused to thermally shrink, the stopper cap is sealed to the syringe. When the syringe is used as an injector, the label and the stopper cap are removed from the syringe, and an injection needle is mounted into the opening of the tip portion of the syringe. In this case, since cut lines (cuts) such as perforations or irregular cuts are provided in a label of Patent Document 1 along the circumferential direction of the label, the label is ruptured from an intermittent portion including the cut lines by twisting the stopper cap, to perform unsealing, and with a portion (residual portion) of the label being left at the syringe side, the other portion of the label is removed together with the stopper cap from the syringe.

### CITATION LIST

### PATENT DOCUMENT

Patent Document 1: Japanese Patent No. 4586079

According to Patent Document 1, by twisting the stopper cap, the label is ruptured from the intermittent portion including the cut lines, to allow for removal of the stopper cap or mounting of the injection needle. However, in order to generate twisting torque for rupturing the intermittent portion, a user needs to firmly hold the stopper cap and strongly twist the stopper cap (with a great twisting force) if the stopper cap has a small diameter (i.e., the length of an arm thereof is short), which is a burden on the user. Furthermore, in order to generate such twisting torque, while holding the syringe with one hand, the user holds and twists the stopper cap with the other hand, so that both hands are occupied. Thus, this may interfere with other actions such as mounting of the injection needle. In addition, the label may be ruptured from the intermittent portion in the circumferential direction (right-left direction) so as to gradually deviate from the intermittent portion to the obliquely upper or lower side. When the label is ruptured obliquely upward from the intermittent portion so as to deviate from the intermittent portion, the residual portion of the label protrudes at the upper side of the syringe, which is likely to cause a trouble (insertion of the residual portion, etc.) in mounting of the injection needle. Moreover, even if the label is ruptured along the intermittent portion, when the syringe is resealed, it is hard to confirm the unsealed state through a glance at the ruptured line in the circumferential direction, and thus it is difficult to find improper unsealing or mistaken unsealing.

### SUMMARY OF INVENTION

### PROBLEMS TO BE SOLVED BY THE INVENTION

An object of the invention is to provide a syringe label which allows for easy unsealing along an intermittent portion and with which an unsealed state is easily confirmed, and a drug solution-filled syringe using the label.

### SOLUTION TO THE PROBLEMS AND EFFECTS OF THE INVENTION

To achieve the above-described object, a syringe label according to the invention is a syringe label which is wrapped around peripheries of both a syringe filled with a drug solution and a projection portion of a shaft-shaped stopper cap mounted in an opening of the syringe, to seal the syringe and the stopper cap. In a wrapped state, a rupture-intended line is formed so as to continuously extend in a predetermined direction, has an intermittent portion including cut lines formed in a penetrating manner in a thickness direction, and intersects with an edge facing the syringe at a predetermined position (e.g., two locations) in a circumferential direction, and by pushing the projection portion of the stopper cap near the rupture-intended line in a predetermined direction, the intermittent portion is ruptured to perform unsealing.

In such a syringe label, the intermittent portion is ruptured, for example, by applying a pushed-over force to the stopper cap in a direction crossing the syringe longitudinal direction or by applying a pushing-out force to the stopper cap in a direction in which the stopper cap is pulled out from the syringe, and it is unnecessary to apply a great twisting force to the stopper cap even if the stopper cap has a small diameter. Thus, it is possible to reduce a burden on a user. In addition, while the syringe is held with one hand, it is possible to push the stopper cap with the thumb of the same hand. Thus, it is also possible to increase the working efficiency by performing rupture of the intermittent portion (breaking of the label) and removal of the stopper cap with one hand and performing mounting of an injection needle or the like with the other hand. Moreover, by pushing the projection portion of the stopper cap near the rupture-intended line, the label does not protrude at the upper side of the syringe after being ruptured, which is less likely to cause a trouble in mounting of the injection needle or the like. Furthermore, since the rupture-intended line intersects with the edge facing the syringe, as compared to the circumferential direction (right-left direction), even in a state where resealing is performed after the intermittent portion is ruptured, it is easy to grasp the unsealed state, and it is possible to easily find improper unsealing or mistaken unsealing.

The intermittent portion is composed of a cut line portion (cut portion) and a cut line non-formed portion (label portion), and cut lines (cuts) having unequal lengths and cut lines having substantially equal lengths (e.g., the difference between the maximum length and the minimum length is within 10%) may be referred to as "irregular cuts" and "perforations", respectively, to be distinguished from each other. The rupture-intended line may include, in addition to the intermittent portion, an easily-cut portion (a portion having easy cuttability such as a linearly cuttable film) and an unsealing designation portion (a cutout indicating an unsealing start position such as a V notch).

In particular, desirably, at least a portion of the stopper cap has flexibility, and when, by pushing a peripheral surface of the projection portion of the stopper cap near the rupture-intended line in a direction from an outer side to an inner side, the stopper cap is pushed over (e.g., on the principle of leverage in a direction crossing the syringe longitudinal direction) with, as a fulcrum, a side opposite in the circumferential direction to a side at which a pushing force acts, the intermittent portion located at the side at which the pushing force acts is ruptured due to elasticity (bending) of the flexible stopper cap, to perform unsealing.

There is an advantage that: the flexibility of the stopper cap can be used to rupture the intermittent portion with a relatively small pushing force such as a force of pushing over on the principle of leverage; and it is easy to confirm the unsealed state, since the ruptured line is located at the near side.

Also in order to achieve the above-described object, a drug solution-filled syringe according to the invention is a drug solution-filled syringe in which the syringe is filled with the drug solution, the stopper cap is mounted in the opening of the syringe, and the syringe is sealed by the syringe label described above.

By using the label which allows for easy unsealing along the intermittent portion and with which an unsealed state is easily confirmed as described above, it is possible to use the drug solution-filled syringe as an injector which is excellent in safety and operability.

### BRIEF DESCRIPTION OF THE DRAWINGS

FIG. 1A is a front cross-sectional view showing a schematic structure of a drug solution-filled syringe according to the invention.
FIG. 1B is a front cross-sectional view showing a form of the drug solution-filled syringe in FIG. 1A in use.
FIG. 2 is a front view showing one embodiment of the label according to the invention in a developed state.
FIG. 3A is an explanatory back view showing an example of a sealing procedure with the label in FIG. 2.
FIG. 3B is a plan view at end of the sealing in FIG. 3A.
FIG. 4 is an enlarged view of the label in a developed state in FIG. 3A.
FIG. 5 is an enlarged view of the label in a wrapped state in FIG. 3A.
FIG. 6A is an explanatory side view showing an example of an unsealing procedure with the label in FIG. 2.
FIG. 6B is a partial front view at start of pushing in FIG. 6A.
FIG. 7A is an explanatory side view showing another example of the unsealing procedure with the label in FIG. 2.
FIG. 7B is a partial front view at start of pushing in FIG. 7A.
FIG. 8 is a front view showing another embodiment of the label according to the invention in a developed state.
FIG. 9 is a front view showing still another embodiment of the label according to the invention in a developed state.
FIG. 10A is a front view showing still another embodiment of the label according to the invention in a developed state.
FIG. 10B is a front view showing still another embodiment of the label according to the invention in a developed state.

### DESCRIPTION OF EMBODIMENTS

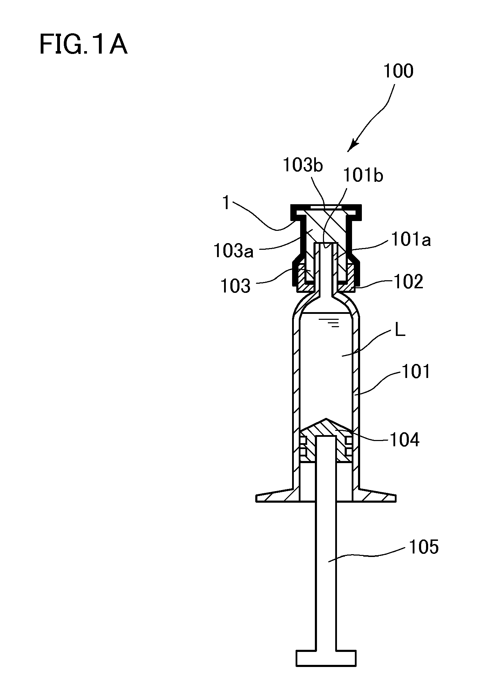

Hereinafter, modes for carrying out the invention will be described with reference to embodiments shown in the drawings. FIG. 1A schematically shows a drug solution-filled syringe according to the invention. To a tip portion 101a of a syringe 101 (injector body) filled with a drug solution L, a cylindrical Luer-Lock adapter (hereinafter, also referred to as adapter) 102 having a larger diameter than the tip portion 101a is fixedly attached. A cylindrical portion of a rubber stopper cap 103 having flexibility in the entirety thereof is fitted between the tip portion 101a of the syringe 101 and the adapter 102, and the stopper cap 103 axially projects longer than the tip portion 101a, so that a projection portion 103a of the stopper cap 103 closes an opening 101b of the tip portion 101a.

A shrink label (hereinafter, also referred to merely as label) 1 as a syringe label is wrapped around the outer peripheral surfaces of both the tip portion 101a of the syringe 101 (the adapter 102 fixedly attached to the tip portion 101a in this embodiment) and the projection portion 103a of the stopper cap 103. The label 1 is composed of a heat-shrinkable resin film, and also covers a portion of a top surface 103b of the projection portion 103a. When the label 1 thermally shrinks in this state, the syringe 101 and the stopper cap 103 are sealed to obtain a drug solution-filled syringe 100. Reference character 104 denotes a bottom stopper, and reference character 105 denotes a plunger rod.

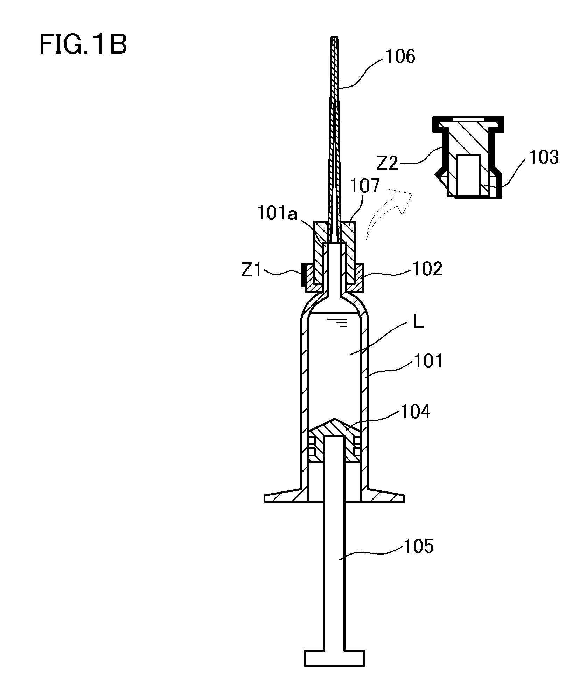

FIG. 1B shows a form of use as an injector. The label 1 is ruptured along a later-described rupture-intended line (intermittent portion) to perform unsealing, a residual area Z1 of the label 1 remains at the tip portion 101a of the syringe 101 (actually at the adapter 102), and a separation area Z2 obtained by excluding the residual area Z1 from the label 1 is separated together with the stopper cap 103 from the tip portion 101a. Thus, the stopper cap 103 is removed from the syringe 101, and an injection needle 106 is mounted to the tip portion 101a. A needle holding portion 107 is fixed to a base portion of the injection needle 106, and instead of the stopper cap 103, a cylindrical end portion of the needle holding portion 107 is fitted (or screwed) between the tip portion 101a of the syringe 101 and the adapter 102.

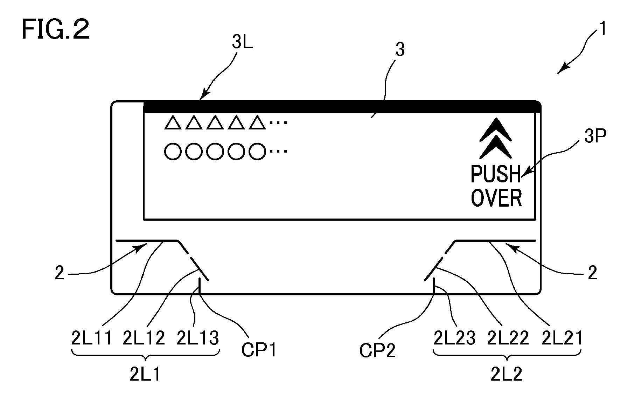

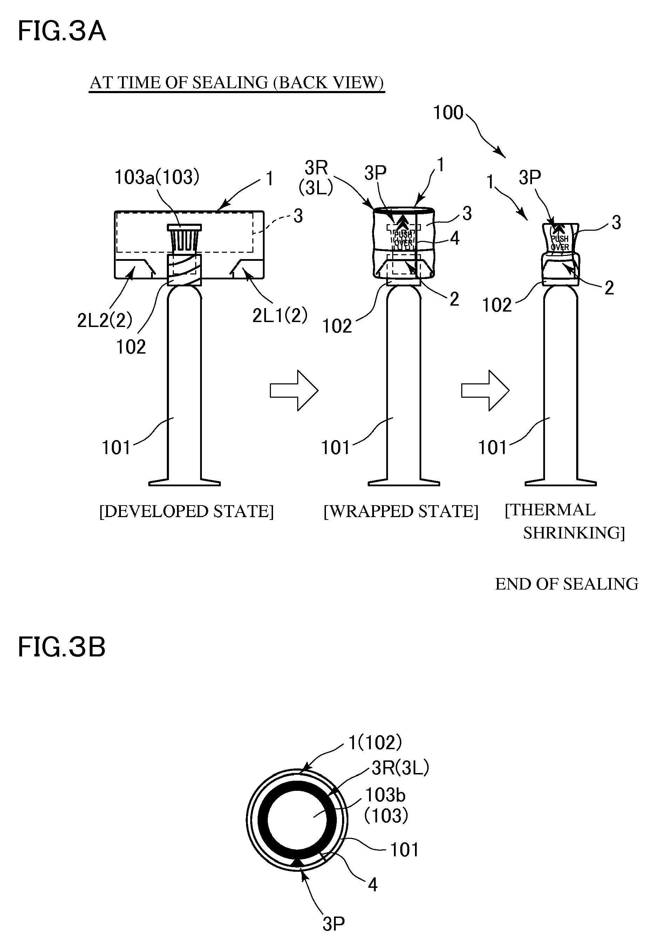

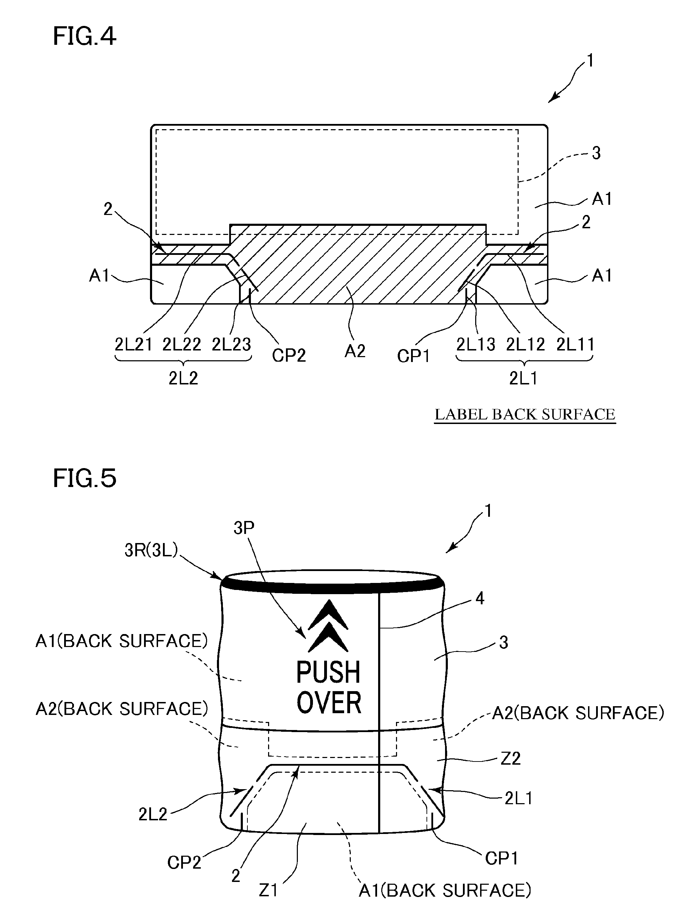

FIG. 2 shows a front view of the label used in FIG. 1A in a developed state, and FIGS. 3A and 4 each show a back view of the label in a developed state. FIG. 3A shows a sealing procedure with the label in FIG. 2, FIG. 4 is an enlarged view of the label in the left-side diagram in FIG. 3A, and FIG. 5 is an enlarged view of the label in the middle diagram in FIG. 3A. In FIGS. 2, 3A, and 4, the label 1 exhibits a rectangular shape which is long in a right-left direction which corresponds to a circumferential direction in which the label is wrapped around the tip portion 101a of the syringe 101 (the adapter 102) and the projection portion 103a of the stopper cap 103. Therefore, the upper side of the label 1 is an edge facing the stopper cap 103, and the lower side of the label 1 is an edge facing the syringe 101. In addition, during wrapping of the label 1, the label 1 is caused to extend around the syringe 101 and the stopper cap 103, and both right and left end portions are overlapped with each other (see FIG. 5).

As shown in FIG. 2 (the front view in the developed state) and FIG. 4 (the back view in the developed state), in a developed state prior to wrapping, a first intermittent portion 2L1 and a second intermittent portion 2L2 are formed separately at both left and right sides in a lower portion (a site corresponding to the tip portion 101a of the syringe 101) of the label 1. Each of the intermittent portions 2L1 and 2L2 is composed of: a cut line portion which is a cut portion formed in a penetrating manner in the thickness direction; and a cut line non-formed portion which is a label portion. The respective intermittent portions 2L1 and 2L2 include: first cut lines 2L11 and 2L21 which are cuts aligned and extending long in the right-left direction and oblique directions; second cut lines 2L12 and 2L22 which are cuts extending in the oblique directions so as to be shorter than the first cut lines 2L11 and 2L21; and third cut lines 2L13 and 2L23 extending from middle portions of the second cut lines 2L12 and 2L22 in an up-down direction so as to be shorter than the second cut lines 2L12 and 2L22, and the first to third cut lines are formed as irregular cuts having unequal lengths. The left and right ends of the first cut lines 2L11 and 2L21 do not reach the left and right sides of the label 1 in the developed state shown in FIGS. 2 and 4. However, by overlapping the left and right end portions of the label 1 with each other across an overlap line 4 during wrapping, the first cut lines 2L11 and 2L21 are substantially integrated with each other as shown in FIG. 5, to form the trapezoidal rupture-intended line 2 which is continuous.

In a wrapped state shown in FIG. 5, the rupture-intended line 2 (specifically, the lower ends of the third cut lines 2L13 and 2L23) reaches the lower side of the label 1 (the edge facing the syringe 101) to intersect with the lower side at two locations in the circumferential direction (intersection points CP1 and CP2). The residual area Z1 surrounded by the lower side of the label 1 and the rupture-intended line 2 remains at the tip portion 101a of the syringe 101 (the adapter 102), and the separation area Z2 excluding the residual area Z1 is separated together with the stopper cap 103 from the tip portion 101a of the syringe 101 to perform unsealing (see FIGS. 1B, 6A, and 7A). Since only the residual area Z1 of the label 1 remains at the syringe 101 side, the residual area Z1 (further the label 1) does not inhibit operations after unsealing such as mounting of an injection needle.

As shown in FIG. 5, a gluing portion A1 (adhesive layer portion) having adhesiveness to the tip portion 101a of the syringe 101 (the adapter 102) is formed over the substantially entirety of the back surface corresponding to the residual area Z1. Since the substantially entirety of the residual area Z1 is the gluing portion A1, the residual area Z1 which remains at time of unsealing is allowed to be attached to the syringe 101 side without falling off.

As shown in FIGS. 4 and 5, a non-gluing portion A2 (non-adhesive layer portion) not having adhesiveness is formed on a part of the back surface corresponding to the separation area Z2. Specifically, the non-gluing portion A2 occupies a large part of a lower half of the label 1 at the side (the back side) in the circumferential direction opposite to the residual area Z1 located at the near side in the wrapped state, and also includes portions above and below the rupture-intended line 2 with a predetermined width. The non-gluing portion A2 is, for example, a portion to which a pressure-sensitive adhesive is not applied, or a portion in which a coating layer not having adhesiveness is laminated on an adhesive layer, and thus it is easy to separate the non-gluing portion A2 from the syringe 101 at time of unsealing.

As shown in FIG. 4, the gluing portion A1 is also formed on the substantially entirety of the back surface corresponding to a later-described display portion 3 (i.e., the back surface of a substantially upper half of the label 1), and thus allows the separation area Z2 separated from the syringe 101 at time of unsealing, to be attached to the stopper cap 103 side.

Therefore, at time of unsealing shown in FIG. 1B, 6A, or 7A, a portion of the gluing portion A1 that corresponds to the residual area Z1 remains at the tip portion 101a of the syringe 101 (the adapter 102). Meanwhile, the non-gluing portion A2 is separated together with the stopper cap 103 from the tip portion 101a of the syringe 101, a portion of the gluing portion A1 (a portion of the separation area Z2) that corresponds to the display portion 3 is attached to the stopper cap 103, and thus the entire separation area Z2 is removed together with the stopper cap 103.

At time of unsealing, the intersection points CP1 and CP2 shown in FIG. 5, etc. are rupture start points on the rupture-intended line 2. However, as shown in FIGS. 2 and 4, in the case of this embodiment, rupture progresses also from the first cut lines 2L11 and 2L21 which are integrated in the right-left direction of the label 1, and thus it is possible to easily perform unsealing even with a relatively small pushing force.

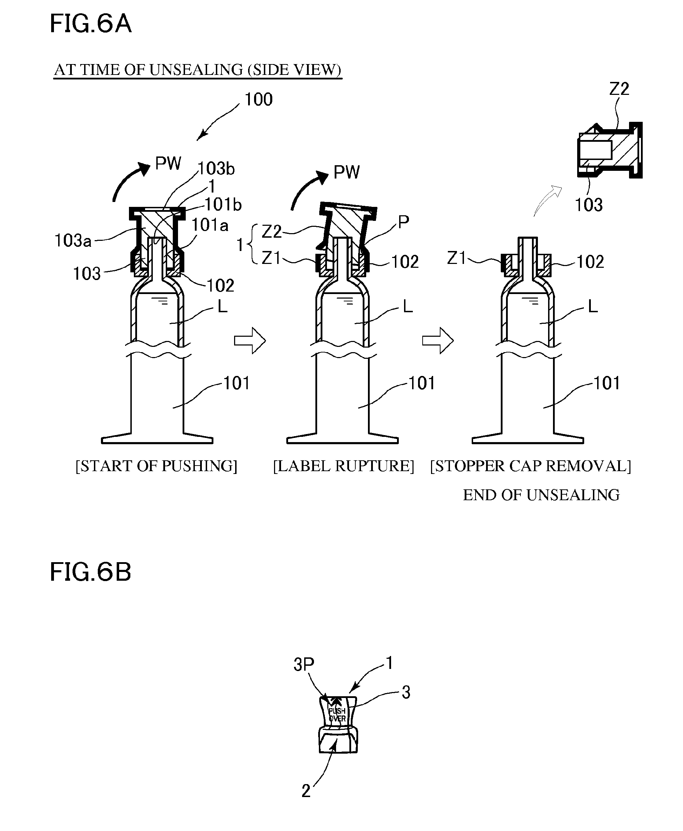

An unsealing procedure shown in FIGS. 6A and 6B will be described. Near (slightly above) the rupture-intended line 2, the peripheral surface of the projection portion 103a of the stopper cap 103 is pushed in a direction from the outer side to the inner side (an arrow direction). At that time, the stopper cap 103 is pushed over on the principle of leverage in a direction (oblique direction) crossing the syringe longitudinal direction (the up-down direction), with, as a fulcrum P, the side(the back side) opposite in the circumferential direction to the side (the near side) at which a pushing force PW acts, and the intermittent portions 2L1 and 2L2 (see FIG. 5) located at the side (the near side) at which the pushing force PW acts are ruptured due to the elasticity (bending) of the flexible stopper cap 103. Then, the residual area Z1 remains at the syringe 101 side, and the separation area Z2 is separated together with the stopper cap 103 from the syringe 101 to perform unsealing.

As described above, even if the stopper cap 103 has a small diameter, it is unnecessary to apply a great twisting force to the stopper cap 103, and rupture of the intermittent portions 2L1 and 2L2 (breaking of the label 1) and removal of the stopper cap 103 can be performed even with one hand by pushing the stopper cap 103. In addition, by pushing the projection portion 103a of the stopper cap 103 above the rupture-intended line 2, since the residual area Z1 does not protrude at the upper side of the syringe 101 and the rupture-intended line 2 intersects with the lower side of the label 1, it is easy to grasp the unsealed state through the intermittent portions 2L1 and 2L2. Furthermore, there is an advantage that: the flexibility of the stopper cap 103 can be used to rupture the intermittent portions 2L1 and 2L2 with a relatively small pushing force PW; and it is easy to confirm the unsealed state, since the intermittent portions 2L1 and 2L2 are located at the near side.

Referring back to FIG. 5, above the rupture-intended line 2 which is continuous in the wrapped state (at the stopper cap 103 side), the display portion 3 is provided which displays the drug solution L with which the syringe 101 is filled, the efficacy thereof, an unsealing method, and the like. The display portion 3 occupies the substantially upper half of the label 1 (see FIG. 2), and the display portion 3 includes: a pushing direction display 3P in which a direction in which the projection portion 103a of the stopper cap 103 is pushed is represented with characters "push over", an upward arrow, etc.; and a line display 3L represented with one thick solid line along the upper side of the label 1 over the substantially entire width in the right-left direction which is a direction perpendicular to the pushing direction display 3P. Since the pushing direction display 3P is located at the projection portion 103a of the stopper cap 103 which is a location which is actually pushed, it is possible to prevent erroneous operation. In addition, in the wrapped state (and at end of sealing after thermal shrinking), the line display 3L forms a circular ring 3R at the top surface 103b of the stopper cap 103 (the projection portion 103a) (see FIG. 3B), and thus normal end of sealing can be confirmed, for example, by capturing an image of (detecting) the circular ring 3R with a detection device (not shown) such as a camera which monitors the top surface 103b from above.

As is obvious also from FIG. 2, FIG. 4, etc., the cut width of the first cut line 2L21 of the second intermittent portion 2L2 is set to be longer than that of the first cut line 2L11 of the first intermittent portion 2L1, in consideration of an overlap margin of the label 1, and the pushing direction display 3P is located above (at the stopper cap 103 side of) the cut line 2L21 having a long cut width. In overlapping both end portions of the label 1 with each other across the overlap line 4, the longer cut line 2L21 is located on (at the near side of) the shorter cut line 2L11 (see FIG. 5).

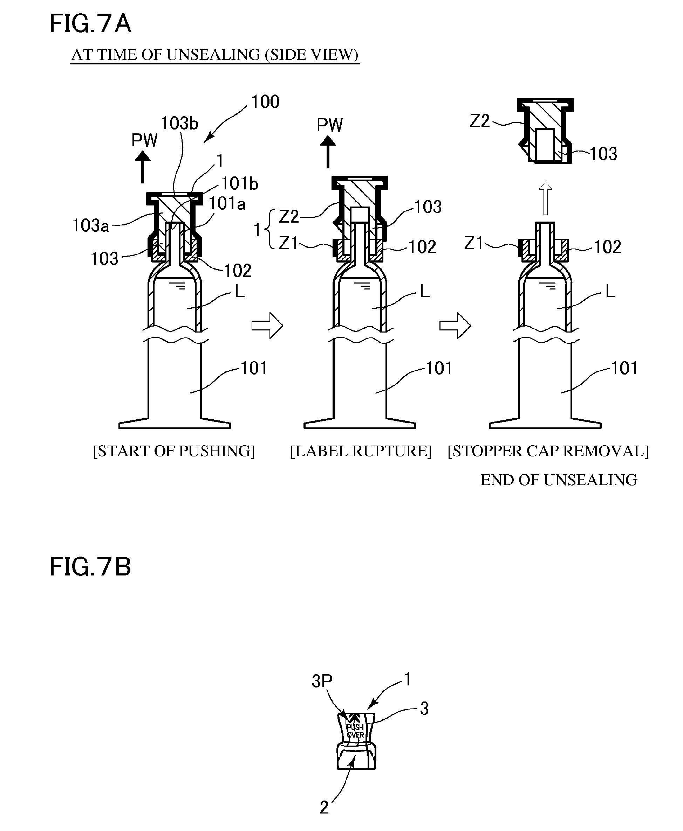

Next, in the case where the stopper cap 103 is made of a material which is relatively hard and difficult to bend (e.g., a synthetic resin), unsealing may be performed through a procedure shown in FIGS. 7A and 7B instead of FIGS. 6A and 6B. Near (slightly above) the rupture-intended line 2, the projection portion 103a of the stopper cap 103 is pushed upward (in an arrow direction). At that time, the stopper cap 103 is pushed out upward by a pushing force PW, so that the intermittent portions 2L1 and 2L2 (see FIG. 5) located at the side (the near side) at which the pushing force PW acts are ruptured. Then, the residual area Z1 remains at the syringe 101 side, and the separation area Z2 is separated together with the stopper cap 103 from the syringe 101 to perform unsealing.

As described above, even if the stopper cap 103 is made of a relatively-hard material, it is unnecessary to apply a twisting force to the stopper cap 103, and rupture of the intermittent portions 2L1 and 2L2 (breaking of the label 1) and removal of the stopper cap 103 can be performed even with one hand by pushing the stopper cap 103. In addition, by pushing the projection portion 103a of the stopper cap 103 above the rupture-intended line 2, since the residual area Z1 does not protrude at the upper side of the syringe 101 and the rupture-intended line 2 intersects with the lower side of the label 1, it is easy to grasp the unsealed state even in a state where resealing is performed after the intermittent portions 2L1 and 2L2 are ruptured. Furthermore, there is an advantage that: the intermittent portions 2L1 and 2L2 can be ruptured with a relatively small pushing force PW; and it is easy to confirm the unsealed state, since the intermittent portions 2L1 and 2L2 are located at the near side.

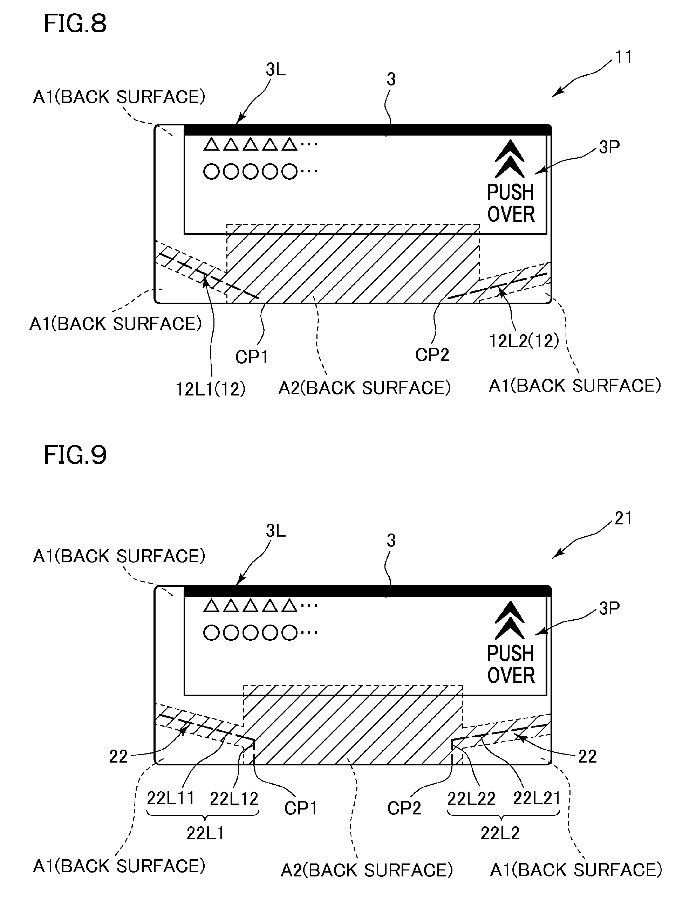

FIG. 8 shows a front view of another embodiment of the label in a developed state. In a developed state prior to wrapping, a first intermittent portion 12L1 and a second intermittent portion 12L2 are formed separately at both left and right sides in a lower portion (a site corresponding to the tip portion 101a of the syringe 101) of a label 11, so as to extend in oblique directions. Each of the intermittent portions 12L1 and 12L2 is composed of: a cut line portion which is a cut portion formed in a penetrating manner in a thickness direction; and a cut line non-formed portion which is a label portion. Each of the intermittent portions 12L1 and 12L2 includes a plurality of (e.g., 6 or 5) cut lines (cuts) aligned in a straight line in the oblique direction, and the respective cut lines are formed as perforations having substantially equal lengths. In the developed state, the left and right ends of the cut lines located at the outermost sides of the respective intermittent portions 12L1 and 12L2 do not reach the left and right sides of the label 11. However, by overlapping the left and right end portions of the label 11 with each other across the overlap line 4 during wrapping, the intermittent portions 12L1 and 12L2 at both sides are integrated into a mountain shape to form a rupture-intended line 12 which is continuous (see FIG. 5). The rupture-intended line 12 (specifically, extensions of the intermittent portions 12L1 and 12L2) reaches the lower side of the label 11 (the edge facing the syringe 101) to intersect with the lower side at two locations in the circumferential direction (intersection points CP1 and CP2).

FIG. 9 shows a front view of still another embodiment of the label in a developed state. In a developed state prior to wrapping, a first intermittent portion 22L1 and a second intermittent portion 22L2 are formed separately at both left and right sides in a lower portion (a site corresponding to the tip portion 101a of the syringe 101) of a label 21. Each of the intermittent portions 22L1 and 22L2 is composed of: a cut line portion which is a cut portion formed in a penetrating manner in a thickness direction; and a cut line non-formed portion which is a label portion. Each intermittent portion 22L1 and 22L2 includes a plurality of (e.g., 5) first cut lines 22L11 and 22L21 (cuts) aligned in a straight line in an oblique direction; and a plurality of (e.g., 2) second cut lines 22L12 and 22L22 (cuts) aligned in a straight line in the up-down direction from the first cut lines 22L11 and 22L21, and the first and second cut lines are formed as perforations having substantially equal lengths. In the developed state, the left and right ends of the first cut lines 22L11 and 22L21 do not reach the left and right sides of the label 21. However, by overlapping the left and right end portions of the label 21 with each other across the overlap line 4 during wrapping, the first cut lines 22L11 and 22L21 at both sides are integrated into a mountain shape to form a rupture-intended line 22 which is continuous (see FIG. 5). The rupture-intended line 22 (specifically, the lower ends of the second cut lines 22L12 and 22L22) reaches the lower side of the label 21 (the edge facing the syringe 101) to intersect with the lower side at two locations in the circumferential direction (intersection points CP1 and CP2).

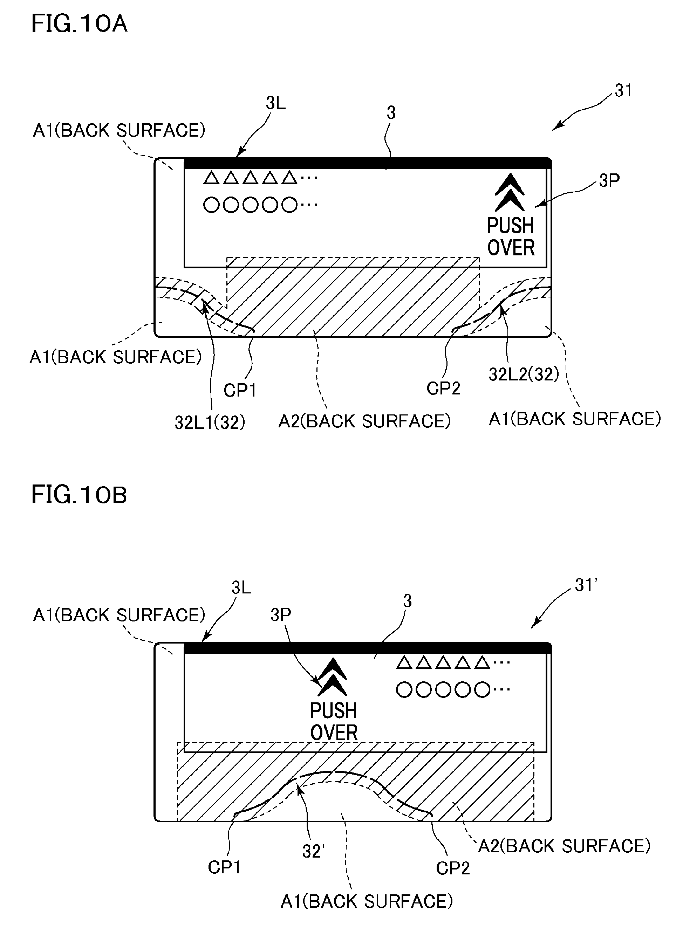

FIGS. 10A and 10B show front views of still other embodiments of the label in a developed state, and show embodiments different from each other. In FIG. 10A, in a developed state prior to wrapping, a first intermittent portion 32L1 and a second intermittent portion 32L2 are formed separately at both left and right sides in a lower portion (a site corresponding to the tip portion 101a of the syringe 101) of a label 31, so as to extend in oblique directions. Each of the intermittent portions 32L1 and 32L2 is composed of: a cut line portion which is a cut portion formed in a penetrating manner in a thickness direction; and a cut line non-formed portion which is a label portion. Each of the respective intermittent portions 32L1 and 32L2 includes a plurality of (e.g., 5) cut lines (cuts) aligned along a curved line in the oblique direction, and the respective cut lines are formed as perforations having substantially equal lengths. In the developed state, the left and right ends of the cut lines located at the outermost sides of the respective intermittent portions 32L1 and 32L2 reach the left and right sides of the label 31. By overlapping the left and right end portions of the label 31 with each other across the overlap line 4 during wrapping, the intermittent portions 32L1 and 32L2 at both sides are integrated into a gentle mountain shape to form a rupture-intended line 32 which is continuous (see FIG. 5). The rupture-intended line 32 (specifically, extensions of the intermittent portions 32L1 and 32L2) reaches the lower side of the label 31 (the edge facing the syringe 101) to intersect with the lower side at two locations in the circumferential direction (intersection points CP1 and CP2).

In FIG. 10B, in a developed state prior to wrapping, an intermittent portion 32' is formed in a unicursal shape in a lower portion (a site corresponding to the tip portion 101a of the syringe 101) of a label 31'. Therefore, in this embodiment, the intermittent portion 32' already forms a rupture-intended line which is continuous, in the developed state (as a matter of course, also in a wrapped state). The intermittent portion 32' is composed of: a cut line portion which is a cut portion formed in a penetrating manner in a thickness direction; and a cut line non-formed portion which is a label portion. The intermittent portion 32' includes a plurality of (e.g., 6) cut lines (cuts) aligned in a gentle mountain shape, and the respective cut lines are formed as perforations having substantially equal lengths. The rupture-intended line (specifically, extensions of the intermittent portion 32') reaches the lower side of the label 31' (the edge facing the syringe 101) to intersect with the lower side at two locations in the circumferential direction (intersection points CP1 and CP2).

Also in the labels 11, 21, 31, and 31', the same advantage as in the case of the label 1 is obtained by applying a pushing-over force to the stopper cap 103 in a direction crossing the syringe longitudinal direction or by applying a pushing-out force to the stopper cap 103 in a direction in which the stopper cap 103 is pulled out from the syringe 101.

The configurations of the gluing portion A1 (adhesive layer portion) and the non-gluing portion A2 (non-adhesive layer portion) of each of the labels 11, 21, 31, and 31' are the same as in the embodiment of the label 1 (see FIGS. 4 and 5) . In addition, the residual area and the separation area are also the same as in the embodiment of the label 1 (see FIG. 5), and are not shown.

In the embodiments described above, only the case has been described in which the adapter 102 is fixedly attached to the tip portion 101a of the syringe 101. However, the invention is also applicable to the case where the adapter 102 is not included or the case where the tip portion 101a is omitted. In addition, the rupture-intended line may have a shape other than the shapes described above. The displayed contents may be highlighted, for example, by applying, to the display portion 3 or the pushing direction display 3P, a color different from that of the other portion.

### DESCRIPTION OF THE REFERENCE CHARACTERS

- 1, 11, 21, 31, 31': shrink label (syringe label)
- 2, 12, 22, 32, 32': rupture-intended line
- 2L1, 12L1, 22L1, 32L1: first intermittent portion
- 2L2, 12L2, 22L2, 32L2: second intermittent portion
- 3: display portion
- 3P: pushing direction display
- 4: overlap line
- 100: drug solution-filled syringe
- 101: syringe (injector body)
- 101a: tip portion
- 101b: opening
- 103: stopper cap
- 103a: projection portion
- L: drug solution
- CP1, CP2: intersection point (rupture start point)
- P: fulcrum
- PW: pushing force
- A1: gluing portion (adhesive layer portion)
- A2: non-gluing portion (non-adhesive layer portion)
- Z1: residual area
- Z2: separation area

## Claims

1. A syringe label which is wrapped around peripheries of both a syringe filled with a drug solution and a projection portion of a shaft-shaped stopper cap mounted in an opening of the syringe, to seal the syringe and the stopper cap, wherein
in a wrapped state, a rupture-intended line is formed so as to continuously extend in a predetermined direction, has an intermittent portion including cut lines formed in a penetrating manner in a thickness direction, and intersects with an edge facing the syringe at a predetermined position in a circumferential direction, and
by pushing the projection portion of the stopper cap near the rupture-intended line in a predetermined direction, the intermittent portion is ruptured to perform unsealing.

2. The syringe label according to claim 1, wherein
at least a portion of the stopper cap has flexibility, and
when, by pushing a peripheral surface of the projection portion of the stopper cap near the rupture-intended line in a direction from an outer side to an inner side, the stopper cap is pushed over with, as a fulcrum, a side opposite in the circumferential direction to a side at which a pushing force acts, the intermittent portion located at the side at which the pushing force acts is ruptured due to elasticity of the flexible stopper cap, to perform unsealing.

3. The syringe label according to claim 1 or 2, wherein
in the wrapped state, the rupture-intended line intersects with the edge facing the syringe at two locations in the circumferential direction, and
a residual area surrounded by the edge facing the syringe and the rupture-intended line remains at the syringe, and a separation area excluding the residual area is separated together with the stopper cap from the syringe to perform unsealing.

4. The syringe label according to claim 3, wherein
a gluing portion having adhesiveness to the syringe is formed on a back surface of the residual area, and a non-gluing portion not having adhesiveness to at least the syringe is formed on a back surface of the separation area so as to include the rupture-intended line, and
at time of unsealing, the gluing portion remains at the syringe, and the non-gluing portion is separated together with the stopper cap from the syringe.

5. The syringe label according to any one of claims 1 to 4, wherein
the intermittent portion is separately formed at both sides in a developed state prior to wrapping, and both end portions of the intermittent portion are overlapped with each other during wrapping to form the continuous rupture-intended line.

6. The syringe label according to any one of claims 1 to 5, wherein
a display portion which displays at least one of the drug solution with which the syringe is filled, an efficacy thereof, and an unsealing method is provided at a stopper cap side with respect to the rupture-intended line which is continuous in the wrapped state, and
the display portion includes a display for a direction in which the projection portion of the stopper cap is pushed.

7. The syringe label according to any one of claims 1 to 6, wherein
the syringe label is wrapped around peripheries of both a Luer-Lock adapter fixedly attached to a tip portion of the syringe and the projection portion of the stopper cap.

8. A drug solution-filled syringe, wherein
the syringe is filled with the drug solution, the stopper cap is mounted in the opening of the syringe, and the syringe is sealed by the syringe label according to any one of claims 1 to 7.
